# FASCICULE DE BREVET EUROPEEN

(11) **EP 3 413 864 B1**
(45) Date de publication et mention de la délivrance du brevet: **25.12.2019**
(21) Numéro de dépôt: 17707614.8
(22) Date de dépôt: 02.02.2017
(51) Int. Cl.: A61K 8/24, A61K 8/25, A61K 8/44, A61Q 11/00, A61K 8/21

(54) **COMPOSITION POUR L'AERO-POLISSAGE**
PULVERZUSAMMENSETZUNG ZUM LUFTPOLIEREN
POWDER COMPOSITION FOR AIR POLISHING

(30) Priorité: 08.02.2016 FR 1650961
(43) Date de publication de la demande: 19.12.2018
(73) Titulaire: Produits Dentaires Pierre Rolland, 33700 Merignac (FR)
(72) Inventeur: MAURAT, Vincent, 33600 Pessac (FR); PIGERON, Clémence, 33200 Bordeaux (FR)
(74) Mandataire: Cabinet Beau de Loménie
(86) Numéro de dépôt international: PCT/FR2017/050225
(87) Numéro de publication internationale: WO 2017/137680

(56) Documents cités:
- WO-A1-2013/191903
- FR-A1- 2 572 925
- US-A- 4 623 536
- US-B1- 6 648 644
- DATABASE GNPD [Online] MINTEL; août 2014 (2014-08), "Anti-inflammation and whitening Tooth powder", XP002764042, Database accession no. 2622061
- DATABASE GNPD [Online] MINTEL; janvier 2009 (2009-01), "Tooth powder", XP002764043, Database accession no. 1037294
- DATABASE GNPD [Online] MINTEL; avril 2004 (2004-04), "Tooth Stain Remover", XP002764044, Database accession no. 264237

## Description

### Arrière-plan de l'invention

L'invention concerne une nouvelle composition pour l'aéro-polissage de la surface des tissus dentaires durs et son utilisation dans un procédé d'aéro-polissage.

Les poudres destinées à l'aéro-polissage dentaire sont généralement à base de glycine, carbonate de calcium ou bicarbonate de sodium. On connaît, en particulier, une poudre à base de glycine mise au point par la société 3M ESPE et disponible sous la référence « Clinpro™ Prophy Powder » utilisée en sous-gingival et une poudre à base de bicarbonate de sodium commercialisée par la société SATELEC sous la référence « Air'n'go Classic® » utilisée en supra-gingival.

Il existe toutefois un besoin pour améliorer les traitements d'aéro-polissage existants en permettant de fonctionnaliser davantage ces traitements sans pour autant conduire à un rallongement significatif du temps de traitement.

Il existe en particulier un besoin pour disposer d'un traitement d'aéro-polissage amélioré permettant en outre d'effectuer une action thérapeutique efficace sur la dent traitée sans conduire à un rallongement significatif du temps de traitement.

### Objet et résumé de l'invention

A cet effet, l'invention propose, une composition pour l'aéro-polissage de la surface des tissus dentaires durs comprenant au moins : - une première poudre abrasive apte à réaliser un polissage des tissus dentaires durs, la première poudre comportant du bicarbonate de sodium, du carbonate de calcium, de la glycine, un silicate, de la silice, de l'hydroxyde de silicium, du carbure de silicium, de la pierre ponce pulvérisée, de la poudre de diamant, de l'oxyde d'aluminium, de l'hydroxyde d'aluminium ou un mélange de ces composés, - une deuxième poudre d'un gélifiant choisi parmi : les poloxamères, la cellulose d'hydroxypropylméthyle, la cellulose de méthyle, les carraghénanes et leurs mélanges, et - une troisième poudre d'un agent de traitement dentaire, l'agent de traitement dentaire étant un agent thérapeutique ou un agent de coloration.

Les tissus dentaires durs désignent l'émail, la dentine et le cément.

La composition selon l'invention est remarquable en ce qu'elle permet, d'une part, de réaliser le traitement d'aéro-polissage du tissu dentaire dur et, d'autre part, de former grâce à la présence de la deuxième poudre de gélifiant un gel actif renfermant l'agent de traitement et permettant de réaliser une action prolongée, notamment thérapeutique, sur la dent traitée. Plus précisément et comme il sera détaillé plus bas, la composition selon l'invention permet, lorsqu'elle est projetée par l'aéro-polisseur à relativement faible distance du tissu traité, de réaliser le polissage recherché et de former le gel actif lorsque l'aéro-polisseur est éloigné de la dent traitée. Ainsi, grâce à la composition selon l'invention, il est possible de réaliser, à l'aide de la même composition et du même outil d'aéro-polissage, deux actions successives la première étant le polissage d'un tissu dentaire dur et la deuxième étant le traitement d'un tissu dentaire par un agent de traitement présent dans le gel actif formé. Le gel actif formé peut permettre de traiter des tissus dentaires durs et/ou des tissus dentaires mous. La formation du gel actif est avantageuse afin de permettre à l'agent de traitement dentaire d'agir sur une durée relativement longue. La composition selon l'invention permet donc avantageusement de fonctionnaliser davantage le traitement d'aéro-polissage dentaire classique en lui associant tout type de traitement dentaire permis par le dépôt du gel sans pour autant complexifier le traitement ou rallonger sa durée.

Le gélifiant peut être un composé organique. En variante, le gélifiant est un composé inorganique.

Dans un exemple de réalisation, le gélifiant peut être choisi parmi : les poloxamères, les carbomères, les polyvinylpyrrolidones, les polyéthylène glycol, l'alcool polyvinylique, les extraits d'algue, les extraits de fruits, la gélatine, les gommes de graines végétales, les exsudats de plantes, la cellulose ou les dérivés de cellulose, les exsudats de microorganismes, les esters d'acide gras de polyoxyéthylène, les esters d'acide gras de polyoxyéthylènesorbitan, les argiles et leurs mélanges.

Le gélifiant de type poloxamère peut être un copolymère à blocs polyéthylène glycol (PEG) - polypropylène glycol (PPG) - polyéthylène glycol.

Le gélifiant de type extrait d'algue peut, par exemple, être choisi parmi : l'agar-agar, les carraghénanes, les alginates, l'acide alginique ou les furcelleranes. Le gélifiant de type alginate peut, par exemple, être choisi parmi : l'alginate de sodium, l'alginate de potassium, l'alginate d'ammonium, l'alginate de calcium ou l'alginate de propane-1,2-diol.

Le gélifiant de type extrait de fruit peut, par exemple, être la pectine.

Le gélifiant de type gomme de graine végétale peut par exemple être choisi parmi : la gomme de caroube, la gomme de guar ou la gomme d'avoine.

Le gélifiant de type exsudat de plante peut par exemple être choisi parmi : la gomme arabique, la gomme karaya ou la gomme adragante.

Le gélifiant de type dérivé de cellulose peut par exemple être choisi parmi : la cellulose de méthyle, la cellulose d'éthyle, la cellulose d'hydroxypropyle, la cellulose d'hydroxypropylméthyle, la cellulose de méthyléthyle ou la cellulose de carboxyméthyle.

Le gélifiant de type exsudat de micro-organisme peut par exemple être choisi parmi : la gomme de xanthane ou la gomme gellane.

Le gélifiant de type ester d'acide gras de polyoxyéthylène peut par exemple être le stéarate de polyoxyéthylène.

Le gélifiant de type ester d'acide gras de polyoxyéthylènesorbitan peut par exemple être choisi parmi : le monolaurate de polyoxyéthylène-20-sorbitan, le monooléate de polyoxyéthylène-20-sorbitan, le monopalmitate de polyoxyéthylène-20-sorbitan, le monostéarate de polyoxyéthylène-20-sorbitan ou le tristéarate de polyoxyéthylène-20-sorbitan.

Le gélifiant de type argile peut par exemple être choisi parmi : le kaolin, les aluminosilicates de magnésium, les bentonites ou l'hectorite.

De préférence, le gélifiant est choisi parmi : les poloxamères, la cellulose d'hydroxypropylméthyle, les carraghénanes et leurs mélanges.

Dans un exemple de réalisation, la deuxième poudre peut être présente dans la composition en une teneur massique comprise entre 0,5% et 80%, par exemple entre 0,5% et 50%.

Dans un exemple de réalisation, la taille moyenne des grains de la deuxième poudre peut être comprise entre 0,5 µm et 400 µm.

Sauf mention contraire, on désigne par « taille moyenne » la dimension donnée par la distribution granulométrique statistique à la moitié de la population, dite D50.

Les particules abrasives de la première poudre peuvent présenter une dureté Mohs supérieure ou égale à 1, de préférence comprise entre 1 et 5, de préférence encore entre 1 et 3.

Dans un exemple de réalisation, la première poudre peut comporter du bicarbonate de sodium, du carbonate de calcium, de la glycine, un silicate, de la silice, de l'hydroxyde de silicium, du carbure de silicium, de la pierre ponce pulvérisée, de la poudre de diamant, de l'oxyde d'aluminium, de l'hydroxyde d'aluminium ou un mélange de ces composés.

La première poudre peut être une poudre de particules organiques, ces particules pouvant être hydrosolubles. De préférence, la première poudre peut comporter du bicarbonate de sodium, du carbonate de calcium, de la glycine ou un mélange de tels composés.

Dans un exemple de réalisation, la première poudre peut être présente dans la composition en une teneur massique comprise entre 15% et 98%, par exemple entre 50% et 98%.

Dans un exemple de réalisation, la taille moyenne des grains de la première poudre peut être comprise entre 5 µm et 500 µm.

De préférence, l'agent de traitement dentaire peut être un agent thérapeutique.

L'agent thérapeutique peut être un agent antiseptique, un agent anti-inflammatoire, un agent anesthésique, un agent désensibilisant, un agent reminéralisant, un agent astringent ou un mélange de tels agents.

De préférence, l'agent désensibilisant peut être choisi parmi le fluorure de sodium, le nitrate de potassium, l'acide oxalique, le fluorure d'étain ou leurs mélanges.

De préférence, l'agent reminéralisant peut être choisi parmi le phosphate de calcium, le chlorure de calcium, le nitrate de calcium, le phosphate de sodium, le phosphate tricalcique, le silicate tricalcique, l'hydroxyapatite, les verres silicates ou leurs mélanges.

De préférence, l'agent antiseptique peut être choisi parmi le chlorhydrate de chlorhexidine, le dichlorhydrate de chlorhexidine, le gluconate de chlorhexidine, le digluconate de chlorhexidine, l'hypochlorite de sodium, les ammoniums quaternaires, les dérivés iodés ou leurs mélanges.

De préférence, l'agent anti-inflammatoire peut être choisi parmi le butoforme, l'acétate de prednisolone, l'acide β-glycyrrhétinique, un anti-inflammatoire non stéroïdien ou leurs mélanges.

De préférence, l'agent anesthésique peut être choisi parmi le chlorhydrate de lidocaïne.

De préférence, l'agent astringent peut être choisi parmi le chlorure d'aluminium.

En variante, l'agent de traitement dentaire peut être un agent de coloration, par exemple un agent de coloration fluorescent. L'agent de coloration peut par exemple être un azurant optique. L'agent de traitement dentaire peut ainsi par exemple être choisi parmi la fluorescéine sodique, le bleu brillant et leurs mélanges.

La troisième poudre peut par exemple présenter une taille moyenne de grains comprise entre 0,5 µm et 500 µm, par exemple comprise entre 0,5 µm et 200 µm.

Avantageusement, la composition peut en outre comporter un agent anti-agglomérant. L'agent anti-agglomérant peut par exemple être choisi parmi la silice colloïdale anhydre, la silice pyrogénée hydrophobe, le phosphate tricalcique et leurs mélanges. L'agent anti-agglomérant peut être sous forme de poudre, les grains de cette poudre pouvant par exemple présenter une taille moyenne comprise entre 0,5 µm et 200 µm.

De préférence, la composition peut comprendre :
- la première poudre en une teneur massique comprise entre 15% et 98%,
- la deuxième poudre en une teneur massique comprise entre 0,5% et 80%,
- la troisième poudre en une teneur massique comprise entre 0,005% et 25%, par exemple comprise entre 0,005% et 20%, et
- un agent anti-agglomérant en une teneur massique comprise entre 0,05% et 10%.

Dans un exemple de réalisation, la composition peut comporter :
- la première poudre en une teneur massique comprise entre 70% et 98%,
- la deuxième poudre en une teneur massique comprise entre 0,5% et 20%,
- la troisième poudre en une teneur massique comprise entre 0,005% et 25%, par exemple entre 0,005% et 20%, et
- un agent anti-agglomérant en une teneur massique comprise entre 0,05% et 10%.

Un tel exemple de formulation est particulièrement adapté au traitement supra-gingival.

Dans un exemple de réalisation, la composition peut comporter :
- la première poudre en une teneur massique comprise entre 15% et 70%,
- la deuxième poudre en une teneur massique comprise entre 20 % et 80 %, par exemple entre 40% et 80%,
- la troisième poudre en une teneur massique comprise entre 0,005% et 25%, par exemple entre 0,005% et 20%, et
- un agent anti-agglomérant en une teneur massique comprise entre 0,05% et 10%.

Un tel exemple de formulation est particulièrement adapté au traitement sous-gingival.

La présente invention vise également un outil d'aéro-polissage dentaire comprenant une buse ainsi qu'un réservoir comprenant une composition telle que décrite plus haut, la buse étant configurée pour projeter sous pression ladite composition entourée d'un jet de liquide.

La présente invention vise également une composition telle que décrite plus haut dont l'agent de traitement est un agent thérapeutique pour son utilisation comme médicament dans le traitement thérapeutique des tissus dentaires.

Dans ce cas, ledit traitement thérapeutique peut comprendre au moins :
- l'aéro-polissage d'un tissu dentaire dur durant lequel la composition entourée d'un jet de liquide est projetée sous pression sur ledit tissu au travers d'une buse d'un outil d'aéro-polissage, la buse étant située à une première distance de la dent traitée durant l'aéro-polissage,
- le positionnement de la buse après l'aéro-polissage à une deuxième distance de la dent traitée supérieure à la première distance, et
- la formation d'un gel actif comprenant l'agent thérapeutique sur la dent traitée par projection sous pression de la composition entourée du jet de liquide au travers de la buse ainsi positionnée à la deuxième distance de la dent traitée.

### Brève description des dessins

D'autres caractéristiques et avantages de l'invention ressortiront de la description suivante de modes particuliers de réalisation de l'invention, donnés à titre d'exemples non limitatifs, en référence aux dessins annexés, sur lesquels :
- les figures 1 et 2 illustrent schématiquement le déroulement d'un exemple de procédé de traitement dentaire supra-gingival mettant en œuvre une composition selon l'invention,
- la figure 3 représente une dent traitée par le procédé illustré aux figures 1 et 2,
- les figures 4 et 5 illustrent schématiquement le déroulement d'un exemple de procédé de traitement dentaire sous-gingival mettant en œuvre une composition selon l'invention,
- la figure 6 représente une dent traitée par le procédé illustré aux figures 4 et 5, et
- la figure 7 est une photographie d'un substrat traité par un exemple de composition selon l'invention montrant à la fois le polissage effectué ainsi que la formation du gel.

### Description détaillée de modes de réalisation

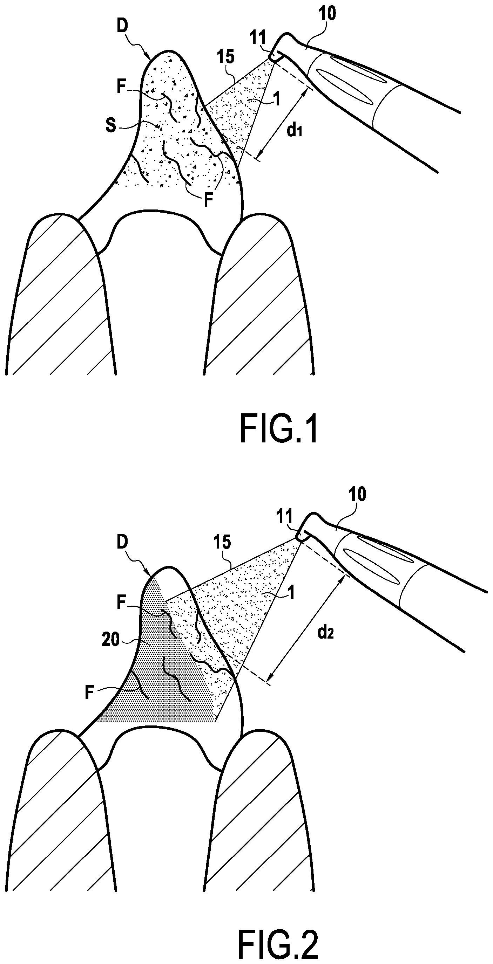

On a représenté à la figure 1 la première étape d'un procédé selon l'invention durant laquelle l'aéro-polissage d'une dent D est effectué à l'aide d'une composition selon l'invention. La dent D est dans l'exemple illustré une incisive. On ne sort bien entendu pas du cadre de l'invention lorsqu'un autre type de dent est traité. La dent D à traiter présente à sa surface une pluralité de fissures F recouvertes d'un dépôt d'une substance S à éliminer, tel qu'un dépôt de plaque dentaire.

Durant cette première étape, un outil d'aéro-polissage dentaire 10 est utilisé afin de projeter sous pression la composition 1 d'aéro-polissage au travers de la buse 11 de l'outil 10. Plus précisément, le jet de composition 1 ainsi qu'un cône de liquide 15 entourant ce jet 1 sont projetés au travers de la buse 11. Le liquide 15 peut par exemple être de l'eau. Un exemple d'outil d'aéro-polissage 10 utilisable est l'aéro-polisseur commercialisé sous la référence AIR-N-GO® par la société ACTEON. La composition 1 projetée comprend au moins, comme décrit plus haut, un mélange des trois constituants suivants : première poudre de particules abrasives, deuxième poudre d'un agent gélifiant et troisième poudre de l'agent de traitement dentaire. La composition 1 peut être pulvérulente. La première poudre peut être différente de la deuxième poudre. La première poudre peut être différente de la troisième poudre. La deuxième poudre peut être différente de la troisième poudre. En variante, la deuxième poudre et la troisième poudre peuvent être identiques. Ce cas est possible dans le cas par exemple où le gélifiant utilisé est un poloxamère lequel peut avoir un effet antiseptique et donc constituer à la fois le gélifiant et l'agent thérapeutique. Comme il sera détaillé plus bas, le gélifiant est configuré pour former un gel sur la dent traitée lors de son mélange avec le liquide projeté par l'outil d'aéro-polissage 10. Avantageusement, la composition 1 peut en outre comporter un agent anti-agglomérant comme décrit plus haut.

L'impact de la composition 1 projetée sur la dent D permet de nettoyer la surface de la dent D et d'éliminer le dépôt S. Durant cette première étape, la buse 11 est située à une distance d₁ relativement faible de la dent D à traiter. De ce fait, le jet de composition 1 impacte la dent D avec une pression relativement élevée ce qui permet d'éliminer le dépôt S présent sur la dent D. La distance d₁ est par exemple inférieure ou égale à 0,5 cm.

Par ailleurs, toujours du fait de cette pression élevée, les constituants de la composition 1 ne se déposent pas sur la dent D plus de quelques secondes durant la première étape et sont évacués de la surface de la dent D avec la substance S juste après impact. Le traitement d'une dent selon cette première étape peut, par exemple, durer au moins une seconde, et par exemple durer environ 3 secondes. Avantageusement, la première poudre peut comporter l'un au moins des composés suivants : bicarbonate de sodium, carbonate de calcium, glycine, un silicate, silice, hydroxyde de silicium, carbure de silicium, une pierre ponce pulvérisée, poudre de diamant, oxyde d'aluminium, hydroxyde d'aluminium ou un mélange de ces composés. Avantageusement, la première poudre peut comporter l'un au moins des composés suivants : bicarbonate de sodium, carbonate de calcium, glycine ou un mélange de tels composés.

Une fois l'étape de polissage effectuée, l'utilisateur éloigne légèrement la buse 11 de la dent D ainsi nettoyée pour la positionner à une distance d₂ et former un gel actif 20 sur la dent D. Dans l'exemple illustré aux figures 1 à 3, la partie de la dent traitée est située au-dessus de la gencive : il s'agit d'un procédé de traitement dentaire supra-gingival. La distance d₂ est par exemple supérieure ou égale à 0,6 cm, par exemple à 1 cm. La réalisation de cette étape est illustrée à la figure 2. Le gel actif 20 est formé suite au mélange entre la deuxième poudre et l'eau projetées par l'outil 10. Ce gel 20 se forme uniquement lorsque la buse 11 est relativement éloignée de la dent D car la pression d'impact de la composition 1 projetée sur la dent D est dans ce cas réduite, ce qui permet aux constituants de la composition 1 de se déposer sur la dent D et au gélifiant de former le gel par mélange avec le liquide. Avantageusement, le gélifiant peut être choisi parmi : les poloxamères, la cellulose d'hydroxypropylméthyle, la cellulose de méthyle, les carraghénanes et leurs mélanges. Le gel actif 20 formé renferme l'agent de traitement dentaire initialement présent dans la composition 1. L'agent de traitement dentaire peut être un agent thérapeutique comme décrit plus haut. Le gel actif 20 permet de réaliser une obturation des fissures F présentes sur la surface de la dent D et d'effectuer un traitement dentaire sur une durée relativement longue, par exemple d'au moins une minute, par exemple de quelques minutes. Le gel actif 20 peut ainsi permettre d'effectuer l'une au moins des actions suivantes : reminéralisation, désensibilisation ou désinfection du tissu dentaire traité. Le gel actif 20 est configuré pour rester au contact de la dent D afin d'effectuer le traitement dentaire recherché. Avantageusement, le gel actif 20 peut être hydrosoluble afin de se dissoudre progressivement dans la bouche du patient une fois le traitement dentaire effectué.

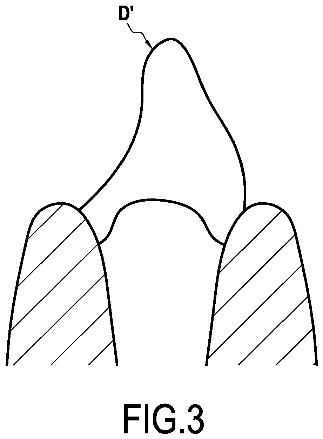

La dent traitée D' obtenue à l'issue des première et deuxième étapes est illustrée à la figure 3. Les fissures F initialement présentes sur la dent D non traitée peuvent être obturées suite au traitement selon l'invention comme illustré.

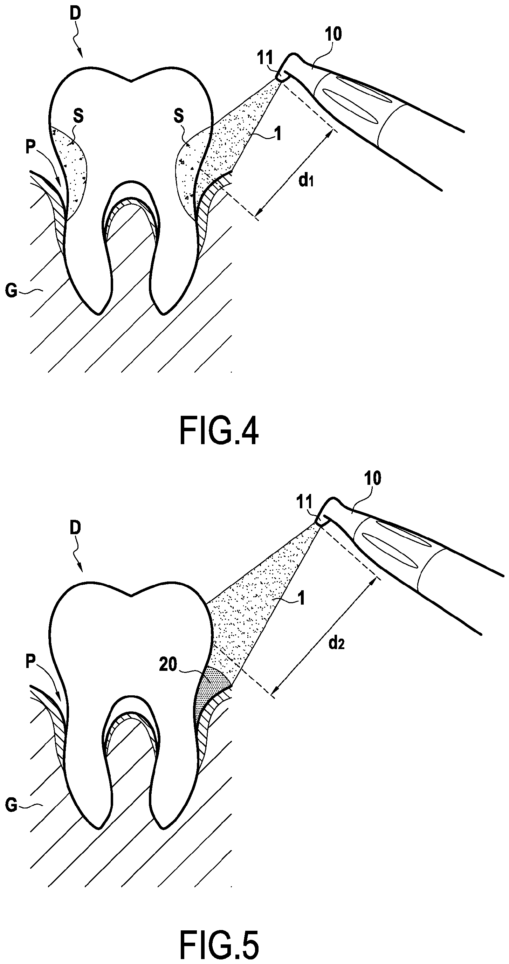

On a représenté aux figures 4 à 6 le traitement d'une dent D atteinte d'une parodontite. Cette dent D est une molaire dans l'exemple illustré. Un aéro-polissage est tout d'abord effectué à l'aide de l'outil 10 et d'une composition telle que décrite plus haut afin d'éliminer la substance S présente à la surface de la dent D (voir figure 4). Une fois ce nettoyage effectué, la buse 11 est ensuite éloignée de la dent traitée afin de former un gel actif 20 sur celle-ci (voir figure 5). Le gel actif 20 est notamment formé dans la poche parondontale P présente entre la gencive rétractée G et la dent D. Le traitement effectué permet de traiter la parodontite et à la gencive de se reconstituer. Le gel formé 20 peut en outre comporter un agent reminéralisant afin de reconstituer l'émail de la dent traitée. On obtient après traitement une gencive saine G' soutenant la dent traitée D'. Pour ce type de traitement sous-gingival on peut avantageusement utiliser une composition présentant une quantité élevée de particules abrasives ayant un faible pouvoir abrasif ou une faible quantité de particules abrasives ayant un fort pouvoir abrasif.

### Exemples

Dans les exemples, toutes les proportions sont exprimées, sauf mention contraire, en pourcentage massique, la température est mesurée en degrés Celsius (°C) et la pression est prise égale à la pression atmosphérique (1 bar), sauf indication contraire.

### Exemple 1

Une composition d'aéro-polissage ayant la formulation détaillée dans le tableau 1 ci-dessous a été réalisée.

**Tableau 1**

| **Formulation** | **%** |
|---|---|
| Bicarbonate de sodium | 81,50 |
| Aérosil R972 | 4,00 |
| Saccharine sodique | 2,00 |
| Fluorure de sodium | 0,50 |
| Nitrate de potassium | 2,50 |
| Nitrate de calcium tetrahydraté | 2,00 |
| Hydrogénophosphate de sodium dodécahydraté | 2,5 |
| Poloxamer | 5,00 |

La composition a été préparée de la manière décrite ci-dessous.

Un premier pré-mélange A est réalisé en ajoutant la moitié de la quantité de bicarbonate de sodium et la moitié de la quantité d'aérosil R972 à un mélange comprenant le nitrate de calcium tétrahydraté et l'hydrogénophosphate de sodium dodécahydraté. L'ensemble est homogénéisé à l'aide d'un mélangeur cubique commercialisé sous la marque FROGERAIS. L'ensemble est ensuite tamisé à 200 µm à l'aide d'un tamis vibrant.

Un second pré-mélange B est réalisé en mélangeant l'autre moitié de la quantité de bicarbonate de sodium, l'autre moitié de la quantité d'aérosil R972, la saccharine sodique, le fluorure de sodium, le nitrate de potassium et le Poloxamer. L'ensemble est homogénéisé à l'aide d'un mélangeur cubique commercialisé sous la marque FROGERAIS. L'ensemble est ensuite tamisé à 200 µm à l'aide d'un tamis vibrant.

Les pré-mélanges A et B sont ensuite homogénéisés afin d'obtenir la composition détaillée au tableau 1 ci-dessus.

Cette composition ainsi obtenue est ensuite fragmentée et tamisée à 200 µm à l'aide respectivement d'un broyeur à marteaux commercialisé par la société Poitemill Forplex et d'un tamis vibrant.

La composition pulvérulente pour l'aéro-polissage obtenue présentait une bonne coulabilité et une granulométrie moyenne D50 comprise entre 70 µm et 90 µm lorsque mesurée avec un appareil type granulomètre laser commercialisé par la société Malvern.

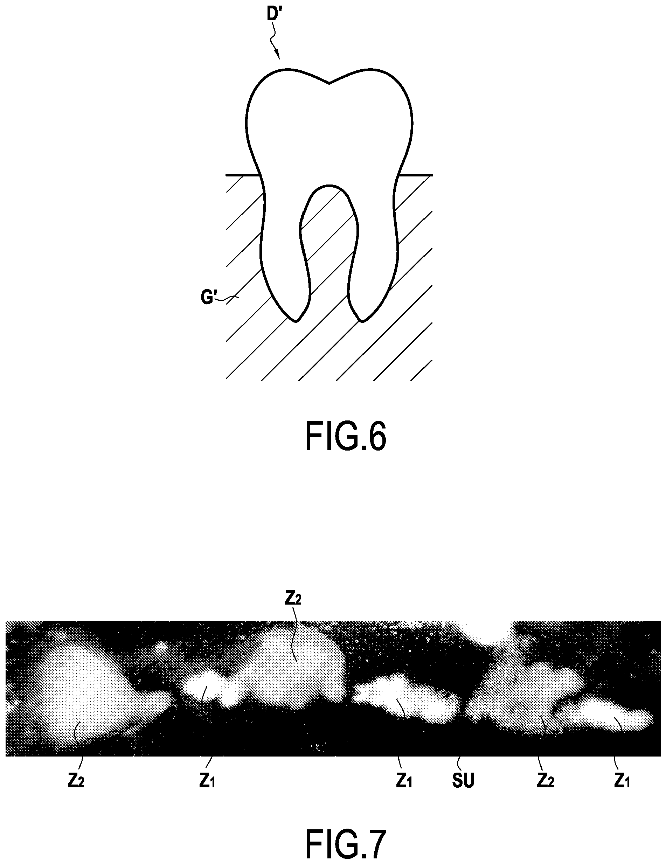

Cette composition permet lorsqu'elle est intégrée dans un outil d'aéro-polissage dentaire de réaliser tout d'abord un polissage supra-gingival puis de former sur la dent traitée un gel reminéralisant et désensibilisant par éloignement de la buse de l'outil d'aéro-polissage. A ce titre, la figure 7 est une photographie d'un substrat SU en carton revêtu d'une couche de peinture noire sur lequel la composition selon l'exemple 1 a été projetée par un outil d'aéro-polissage dentaire. Les zones Z₁ correspondent à des zones « traitées » à courte distance. Ces zones Z₁ ont été polies sans former de gel. On voit que, suite au polissage effectué dans ces zones Z₁, le revêtement de peinture noire a été éliminé, ce qui fait apparaître le substrat sous-jacent, ce dernier apparaissant blanc sur la photographie de la figure 7. Les zones Z₂ correspondent à des zones qui ont été tout d'abord polies, comme les zones Z₁, puis pour lesquelles la buse de l'outil d'aéro-polissage a été éloignée afin de former un gel. Ces zones Z₂ apparaissent grises sur la photographie de la figure 7 du fait de la formation de ce gel.

### Exemple 2

Une composition d'aéro-polissage ayant la formulation détaillée dans le tableau 2 ci-dessous a été fabriquée.

**Tableau 2**

| **Formulation** | **%** |
|---|---|
| Carbonate de calcium | 20,00 |
| Aérosil R972 | 1,00 |
| Saccharine sodique | 1,00 |
| Chlorhydrate de chlorhexidine | 2,50 |
| Nitrate de potassium | 2,50 |
| β - Phosphate tricalcique | 17,00 |
| Carraqhénane | 56,00 |

La composition détaillée au tableau 2 ci-dessus a été obtenue par ajout, dans l'ordre, de la saccharine sodique, du chlorhydrate de chlorhexidine, du nitrate de potassium, du β - phosphate tricalcique, du gélifiant carraghénane et de l'aérosil R972 au carbonate de calcium.

L'ensemble a ensuite été homogénéisé à l'aide d'un mélangeur de type mélangeur par retournement sur potence commercialisé par la société Servolift. La composition obtenue a ensuite été tamisée à l'aide d'un tamis vibrant.

La composition obtenue présentait une granulométrie moyenne entre 40 µm et 60 µm lorsque mesurée à l'aide d'un granulomètre laser de type « Mastersizer 2000 » commercialisé par la société Malvern.

La composition ainsi fabriquée peut être utilisée en supra- ou sous-gingival pour réaliser un polissage puis un gel constituant un « pansement » antiseptique permettant de traiter la poche parodontale.

### Exemple 3

Une composition d'aéro-polissage ayant la formulation détaillée dans le tableau 3 ci-dessous a été fabriquée.

**Tableau 3**

| **Formulation** | **%** |
|---|---|
| Glycine | 60,00 |
| Aérosil R972 | 2,00 |
| Saccharine sodique | 2,00 |
| Arome menthe | 0,50 |
| Chlorhydrate de lidocaïne | 2,50 |
| Hydroxyapatite | 18,00 |
| Methocel E4M | 15,00 |

Cette composition a été fabriquée par broyage de la glycine avec un broyeur à marteaux commercialisé par la société Poitemill Forplex. Puis à la glycine broyée ont été ajoutés, dans l'ordre, la saccharine sodique, l'huile essentielle de menthe poivrée, le chlorhydrate de lidocaïne, l'hydroxyapatite, le Methocel E4M et l'Aérosil R972. La composition a été homogénéisée à l'aide d'un mélangeur en V commercialisé par la société Olsa puis tamisée à 300 µm à l'aide d'un tamis vibrant.

La composition obtenue présentait une granulométrie moyenne comprise entre 15 µm et 35 µm mesurée à l'aide d'un granulomètre laser de type « Mastersizer 2000 » commercialisé par la société Malvern.

La composition ainsi fabriquée permet de réaliser, suite à la formation du gel actif, l'anesthésie des tissus mous dentaires lors d'une application dans une poche parodontale au cours du nettoyage de la poche.

### Exemple 4

Une composition d'aéro-polissage ayant la formulation détaillée dans le tableau 4 ci-dessous a été fabriquée.

**Tableau 4**

| **Formulation** | **%** |
|---|---|
| Glycine | 15,00 |
| Aérosil R972 | 3,00 |
| Saccharine sodique | 2,00 |
| Chlorure d'aluminium | 10,00 |
| Kaolin | 27,70 |
| Huile essentielle de citron jaune | 0,30 |
| Methyl Cellulose | 42,00 |

Un pré-mélange A est réalisé en ajoutant la moitié de la quantité de glycine et la moitié de la quantité de l'aérosil R972 au mélange de chlorure d'aluminium et de kaolin. L'ensemble est homogénéisé à l'aide d'un mélangeur cubique commercialisé sous la marque FROGERAIS puis tamisé à 200 µm à l'aide d'un tamis vibrant.

Un second pré-mélange B est réalisé en mélangeant l'autre moitié de la quantité de glycine, la saccharine sodique, l'huile essentielle de citron jaune, la methyl cellulose et l'autre moitié de la quantité d'Aérosil R972. L'ensemble est homogénéisé à l'aide d'un mélangeur cubique commercialisé par la société Servolift puis tamisé à 200 µm à l'aide d'un broyeur oscillant de la marque Frewitt.

On réalise ensuite l'homogénéisation du pré-mélange A et du pré-mélange B. La composition obtenue est ensuite fragmentée et tamisée à 200 µm à l'aide d'un broyeur conique commercialisé par Frewitt afin d'obtenir la composition détaillée au tableau 4 ci-dessus. Cette composition présentait une bonne coulabilité et une granulométrie moyenne D50 comprise entre 25 µm et 45 µm mesurée avec un appareil type granulomètre laser commercialisé par la société Malvern.

La composition ainsi fabriquée permet de réaliser un polissage sous-gingival et dans un second temps de former, par éloignement de la buse de l'aéro-polisseur, un gel hémostatique permettant de maintenir le sulcus ouvert après rinçage du gel du fait du traitement par le chlorure d'aluminium.

L'expression « compris(e) entre ... et ... » doit se comprendre comme incluant les bornes.

## Revendications

1. Composition pour l'aéro-polissage de la surface des tissus dentaires durs comprenant au moins :
- une première poudre abrasive apte à réaliser un polissage des tissus dentaires durs, la première poudre comportant du bicarbonate de sodium, du carbonate de calcium, de la glycine, un silicate, de la silice, de l'hydroxyde de silicium, du carbure de silicium, de la pierre ponce pulvérisée, de la poudre de diamant, de l'oxyde d'aluminium, de l'hydroxyde d'aluminium ou un mélange de ces composés,
- une deuxième poudre d'un gélifiant choisi parmi : les poloxamères, la cellulose d'hydroxypropylméthyle, la cellulose de méthyle, les carraghénanes et leurs mélanges, et
- une troisième poudre d'un agent de traitement dentaire, l'agent de traitement dentaire étant un agent thérapeutique ou un agent de coloration.

2. Composition selon la revendication 1, la deuxième poudre étant présente dans la composition en une teneur massique comprise entre 0,5% et 80%.

3. Composition selon la revendication 1 ou 2, la taille moyenne des grains de la deuxième poudre étant comprise entre 0,5 µm et 400 µm.

4. Composition selon l'une quelconque des revendications 1 à 3, la première poudre comportant du bicarbonate de sodium, du carbonate de calcium, de la glycine ou un mélange de tels composés.

5. Composition selon l'une quelconque des revendications 1 à 4, la première poudre comprenant de la glycine et la deuxième poudre comprenant un poloxamère.

6. Composition selon l'une quelconque des revendications 1 à 5, la première poudre comprenant du bicarbonate de sodium et la deuxième poudre comprenant un poloxamère.

7. Composition selon l'une quelconque des revendications 1 à 6, la première poudre étant présente dans la composition en une teneur massique comprise entre 15% et 98%.

8. Composition selon l'une quelconque des revendications 1 à 7, la taille moyenne des grains de la première poudre étant comprise entre 5 µm et 500 µm.

9. Composition selon l'une quelconque des revendications 1 à 8, comprenant :
- la première poudre en une teneur massique comprise entre 15% et 98%,
- la deuxième poudre en une teneur massique comprise entre 0,5% et 80%, et
- la troisième poudre en une teneur massique comprise entre 0,005% et 25%, et
- éventuellement un agent anti-agglomérant en une teneur massique comprise entre 0,05% et 10%.

10. Composition selon la revendication 9, comprenant :
- la première poudre en une teneur massique comprise entre 70% et 98%,
- la deuxième poudre en une teneur massique comprise entre 0,5% et 20%, et
- la troisième poudre en une teneur massique comprise entre 0,005% et 25%, et
- éventuellement un agent anti-agglomérant en une teneur massique comprise entre 0,05% et 10%.

11. Composition selon la revendication 9, comprenant :
- la première poudre en une teneur massique comprise entre 15% et 70%,
- la deuxième poudre en une teneur massique comprise entre 20% et 80%, et
- la troisième poudre en une teneur massique comprise entre 0,005% et 25%, et
- éventuellement un agent anti-agglomérant en une teneur massique comprise entre 0,05% et 10%.

12. Composition selon l'une quelconque des revendications 1 à 11, l'agent thérapeutique étant un agent antiseptique, un agent anti-inflammatoire, un agent anesthésique, un agent désensibilisant, un agent reminéralisant, un agent astringent ou un mélange de tels agents.

13. Outil d'aéro-polissage dentaire comprenant une buse ainsi qu'un réservoir comprenant une composition selon l'une quelconque des revendications 1 à 12, la buse étant configurée pour projeter sous pression ladite composition entourée d'un jet de liquide.

14. Composition selon l'une quelconque des revendications 1 à 12 pour la mise en œuvre d'un traitement thérapeutique des tissus dentaires.

## Patentansprüche

1. Zusammensetzung zum Air-Polishing der Oberfläche von harten Zahngeweben, wobei die Zusammensetzung mindestens umfasst:
- ein erstes abrasives Pulver, das dazu geeignet ist, ein Polieren von harten Zahngeweben umzusetzen, wobei das erste Pulver Natriumbicarbonat, Calciumcarbonat, Glycin, Silicat, Siliciumdioxid, Siliciumhydroxid, Siliciumcarbid, pulverisierten Bimsstein, Diamantpulver, Aluminiumoxid, Aluminiumhydroxid oder ein Gemisch dieser Verbindungen umfasst,
- ein zweites Pulver eines Geliermittels, ausgewählt aus: Poloxameren, Hydroxypropylmethylcellulose, Methylcellulose, Carrageenen und ihren Gemischen, und
- ein drittes Pulver eines Zahnbehandlungsmittels, wobei das Zahnbehandlungsmittel ein therapeutisches Mittel oder ein Farbmittel ist.

2. Zusammensetzung nach Anspruch 1, wobei das zweite Pulver in der Zusammensetzung in einem Massengehalt vorliegt, der zwischen 0,5 % und 80 % liegt.

3. Zusammensetzung nach Anspruch 1 oder 2, wobei die durchschnittliche Größe von Körnern des zweiten Pulvers zwischen 0,5 µm und 400 µm liegt.

4. Zusammensetzung nach einem der Ansprüche 1 bis 3, wobei das erste Pulver Natriumbicarbonat, Calciumcarbonat, Glycin oder ein Gemisch derartiger Verbindungen umfasst.

5. Zusammensetzung nach einem der Ansprüche 1 bis 4, wobei das erste Pulver Glycin umfasst und das zweite Pulver ein Poloxamer umfasst.

6. Zusammensetzung nach einem der Ansprüche 1 bis 5, wobei das erste Pulver Natriumbicarbonat umfasst und das zweite Pulver ein Poloxamer umfasst.

7. Zusammensetzung nach einem der Ansprüche 1 bis 6, wobei das erste Pulver in der Zusammensetzung in einem Massengehalt vorliegt, der zwischen 15 % und 98 % liegt.

8. Zusammensetzung nach einem der Ansprüche 1 bis 7, wobei die durchschnittliche Größe von Körnern des ersten Pulvers zwischen 5 µm und 500 µm liegt.

9. Zusammensetzung nach einem der Ansprüche 1 bis 8, umfassend:
- das erste Pulver in einem Massengehalt, der zwischen 15 % und 98 % liegt,
- das zweite Pulver in einem Massengehalt, der zwischen 0,5 % und 80 % liegt, und
- das dritte Pulver in einem Massengehalt, der zwischen 0,005 % und 25 % liegt, und
- gegebenenfalls ein Antiagglomerationsmittel in einem Massengehalt, der zwischen 0,05 % und 10 % liegt.

10. Zusammensetzung nach Anspruch 9, umfassend:
- das erste Pulver in einem Massengehalt, der zwischen 70 % und 98 % liegt,
- das zweite Pulver in einem Massengehalt, der zwischen 0,5 % und 20 % liegt, und
- das dritte Pulver in einem Massengehalt, der zwischen 0,005 % und 25 % liegt, und
- gegebenenfalls ein Antiagglomerationsmittel in einem Massengehalt, der zwischen 0,05 % und 10 % liegt.

11. Zusammensetzung nach Anspruch 9, umfassend:
- das erste Pulver in einem Massengehalt, der zwischen 15 % und 70 % liegt,
- das zweite Pulver in einem Massengehalt, der zwischen 20 % und 80 % liegt, und
- das dritte Pulver in einem Massengehalt, der zwischen 0,005 % und 25 % liegt, und
- gegebenenfalls ein Antiagglomerationsmittel in einem Massengehalt, der zwischen 0,05 % und 10 % liegt.

12. Zusammensetzung nach einem der Ansprüche 1 bis 11, wobei das therapeutische Mittel ein antiseptisches Mittel, ein entzündungshemmendes Mittel, ein Anästhetikum, ein desensibilisierendes Mittel, ein remineralisierendes Mittel, ein Adstringens oder ein Gemisch derartiger Mittel ist.

13. Dentales Air-Polishing-Instrument, umfassend eine Düse sowie ein Reservoir, das eine Zusammensetzung nach einem der Ansprüche 1 bis 12 umfasst, wobei die Düse dazu ausgestaltet ist, die Zusammensetzung, die von einem Flüssigkeitsstrahl umgeben ist, unter Druck zu sprühen.

14. Zusammensetzung nach einem der Ansprüche 1 bis 12 zur Durchführung einer therapeutischen Behandlung von Zahngeweben.

## Claims

1. A composition for air polishing the surface of hard dental tissue, the composition comprising at least:
• an abrasive first powder suitable for polishing hard dental tissue, the first powder comprising sodium bicarbonate, calcium carbonate, glycine, a silicate, silica, silicon hydroxide, silicon carbide, powdered pumice stone, diamond powder, aluminum oxide, aluminum hydroxide, or a mixture of these compounds;
• a second powder of a gelling agent selected from: poloxamers, hydroxypropylmethyl cellulose, methyl cellulose, carrageenans, and mixtures thereof; and
• a third powder of a dental treatment agent, the dental treatment agent being a therapeutic agent or a coloring agent.

2. A composition according to claim 1, the second powder being present in the composition at a content by weight lying in the range 0.5% to 80%.

3. A composition according to claim 1 or claim 2, the mean size of the grains of the second powder lying in the range 0.5 µm to 400 µm.

4. A composition according to any one of claims 1 to 3, the first powder comprising sodium bicarbonate, calcium carbonate, glycine, or a mixture of such compounds.

5. A composition according to any one of claims 1 to 4, the first powder comprising glycine and the second powder comprising a poloxamer.

6. A composition according to any one of claims 1 to 5, the first powder comprising sodium bicarbonate and the second powder comprising a poloxamer.

7. A composition according to any one of claims 1 to 6, the first powder being present in the composition at a content by weight lying in the range 15% to 98%.

8. A composition according to any one of claims 1 to 7, the mean grain size of the first powder lying in the range 5 µm to 500 µm.

9. A composition according to any one of claims 1 to 8, comprising:
• the first powder at a content by weight lying in the range 15% to 98%;
• the second powder at a content by weight lying in the range 0.5% to 80%; and
• the third powder at a content by weight lying in the range 0.005% to 25%; and
• optionally, an anti-caking agent at a content by weight lying in the range 0.05% to 10%.

10. A composition according to claim 9, comprising:
• the first powder at a content by weight lying in the range 70% to 98%;
• the second powder at a content by weight lying in the range 0.5% to 20%; and
• the third powder at a content by weight lying in the range 0.005% to 25%; and
• optionally, an anti-caking agent at a content by weight lying in the range 0.05% to 10%.

11. A composition according to claim 9, comprising:
• the first powder at a content by weight lying in the range 15% to 70%;
• the second powder at a content by weight lying in the range 20% to 80%; and
• the third powder at a content by weight lying in the range 0.005% to 25%; and
• optionally, an anti-caking agent at a content by weight lying in the range 0.05% to 10%.

12. A composition according to any one of claims 1 to 11, the therapeutic agent being an antiseptic agent, an antiinflammatory agent, an anesthetic agent, a desensitizing agent, a remineralizing agent, an astringent agent, or a mixture of such agents.

13. A dental air polishing tool having a nozzle and a tank containing a composition according to any one of claims 1 to 12, the nozzle being configured to spray said composition under pressure surrounded by a jet of liquid.

14. A composition according to any one of claims 1 to 12, for performing therapeutic treatment of dental tissue.
